# EUROPEAN PATENT APPLICATION

(11) **EP 3 915 574 A1**
(43) Date of publication of application: **01.12.2021**
(21) Application number: 20382452.9
(22) Date of filing: 27.05.2020
(51) Int. Cl.: A61K 38/57, A61K 39/395, A61P 29/00, A61P 43/00, A61K 38/20

(54) **METHOD FOR THE TREATMENT OF CYTOKINE RELEASE SYNDROME**

(71) Applicant: Grifols Worldwide Operations Limited, Dublin 22 (IE)
(72) Inventor: TERENCIO ALEMANY, JOSE VICENTE, DUBLIN 22 (IE); BÜSCHER, DIRK, DUBLIN 22 (IE); CARMONA OROZCO, MARIA DEL CARMEN, DUBLIN 22 (IE)
(74) Representative: Durán-Corretjer, S.L.P.

(57) **Abstract**

The present invention refers to methods and compositions for the treatment of Cytokine Release Syndrome (CRS) in a patient in need thereof, comprising administering to the patient a therapeutically effective amount of an inhibitor of interleukin-6 in combination with a therapeutically effective amount of human α-1-proteinase inhibitor (A1AT).

## Description

The present disclosure is related to the field of pharmaceutical products. In particular, the present application refers to methods and compositions for the treatment of Cytokine Release Syndrome (CRS) in a patient in need thereof, the method comprising administering to the patient a therapeutically effective amount of an inhibitor of interleukin-6 (IL-6), such as tocilizumab, and human α-1-proteinase inhibitor (A1AT).

Human α-1-proteinase inhibitor (A1AT) is a plasma protein fraction also known as human α-1-antitrypsin or α-1-antiproteinase. A1AT is the most abundant multifunctional serine proteinase inhibitor in human plasma. It is an acute-phase glycoprotein, and its best characterized target is neutrophil elastase (although other serine proteases like proteinase 3 and cathepsin G have been reported as potential targets).

A1AT is indicated for chronic augmentation and maintenance therapy in individuals with alpha1-proteinase inhibitor deficiency and clinical evidence of emphysema. This is a rare genetic disease often called "genetic COPD", where patients do not generate enough A1AT. This deficiency is associated with lung and liver diseases (e.g. reduced lung function, emphysema, infections...), probably due to the proteinase-antiproteinase imbalance that results in tissue damage caused by the excessive proteinase activity.

In addition to the antiproteinase dependent tissue damage protection, scientific studies published in the last years suggest that A1AT may also play a role as an anti-inflammatory and an immunoregulatory protein. Indeed, preliminary findings in animals have suggested that A1AT could be used for the management of diseases not necessarily related to inherited A1AT deficiency and several clinical trials have been carried out to test the use of A1AT in other indications. Some examples of indications are:
- Diabetes Mellitus type 1
- HIV infection
- Neuromyelitis optica
- Acute Myocardial infarction (AMI)
- Graft vs Host disease (transplantation)
- Pancreatitis
- Lung transplantation rejection
- Cystic fibrosis
- COPD
- Post Cardiac Surgery Systemic Inflammatory Response

On the other hand, tocilizumab is a biologic medication currently approved to treat adults with moderately to severely active rheumatoid arthritis (RA), adults with giant cell arteritis (GCA), and children ages two years old and above with Polyarticular Juvenile Idiopathic Arthritis (PJIA) or Systemic Juvenile Idiopathic Arthritis (SJIA). Biologic medications are proteins designed by humans that affect the immune system. Tocilizumab blocks the inflammatory protein interleukin-6 (IL-6). This improves joint pain and swelling from arthritis and other symptoms caused by inflammation.

Cytokine release syndrome (CRS) is a systemic inflammatory response syndrome (SIRS) that can be triggered by a variety of factors such as infections, biological drugs, and transplantations. CRS occurs when large numbers of immune cells are activated to release inflammatory cytokines, which in turn activate more immune cells in an expanding cycle of inflammation.

When cytokines are released into the circulation, systemic symptoms such as fever, nausea, chills, hypotension, tachycardia, asthenia, headache, rash, scratchy throat, and dyspnea can result. In most patients, the symptoms are mild to moderate in severity and are managed easily. Patients with severe CRS are treated with drugs that counteract the immune response. These medicines include targeted therapies to block specific cytokines, as well as more general immunosuppressive drugs.

The present inventors have surprisingly found a synergistic effect of a composition comprising tocilizumab and A1AT for the treatment of CRS in patients in need thereof, that is, a combined therapy using these two pharmaceutical components will improve the treatment of said condition. After a simulation of the predicted effect on CRS of A1AT in combination with about 3000 approved medicaments, the higher effect (95 %) was combination with tocilizumab.

### SUMMARY

The present disclosure provides methods and compositions for the treatment of CRS in a patient in need thereof. In some embodiments, the method comprises administering to the patient a therapeutically effective amount of an inhibitor of interleukin-6 (IL-6) in combination with a therapeutically effective amount of A1AT.

In some embodiments, the IL-6 inhibitor is tocilizumab.

In some embodiments, the CRS can be mild, moderate or severe.

In some embodiments, the CRS is caused by a viral infection, or by a bacterial infection, or by a fungal infection, or by a protozoal infection, or by a combination thereof.

In some embodiments, the CRS is caused by the SARS-COV-2 virus.

In some embodiments, the composition is administered intravenously.

In some embodiments, tocilizumab can be administered in an amount of about 4 mg/kg to about 8 mg/kg, preferably in an amount of about 5 mg/kg to about 7 mg/kg, at a dose of 400 mg/dose to 800 mg/dose, preferable between 500 mg/dose and 700 mg/dose.

In some embodiments, A1AT can be administered in an amount between 60 mg/kg and 200 mg/kg, preferably between 80 mg/kg and 160 mg/kg, most preferably 120 mg/kg.

The composition of the present invention can be administered at least every 6 hours, or at least every 8 hours, or at least every 12 hours, or at least every 24 hours, or at least every 48 hours, or at least every 72 hours, or at least once a week, or at least once every two weeks. Preferably, the composition is administered twice, one week after the first administration. It is also contemplated that the composition of the present invention is administered as a single dose.

In another aspect, the present invention refers to method and composition for the treatment of Acute Respiratory Distress Syndrome (ARDS) resulting from CRS, the method comprises administering to the patient a therapeutically effective amount of an inhibitor of interleukin-6 (IL-6) in combination with a therapeutically effective amount of A1AT.

In some embodiments, the IL-6 inhibitor is tocilizumab.

In some embodiments, the composition is administered intravenously.

In some embodiments, tocilizumab can be administered in an amount of about 4 mg/kg to about 8 mg/kg, preferably in an amount of about 5 mg/kg to about 7 mg/kg, at a dose of 400 mg/dose to 800 mg/dose, preferable between 500 mg/dose and 700 mg/dose.

In some embodiments, A1AT can be administered in an amount between 60 mg/kg and 200 mg/kg, preferably between 80 mg/kg and 160 mg/kg, most preferably 120 mg/kg.

The composition of the present invention can be administered at least every 6 hours, or at least every 8 hours, or at least every 12 hours, or at least every 24 hours, or at least every 48 hours, or at least every 72 hours, or at least once a week, or at least once every two weeks. Preferably, the composition is administered twice, one week after the first administration. It is also contemplated that the composition of the present invention is administered as a single dose.

In a further aspect, the present invention refers to method and composition for the treatment of Septic Shock, the method comprises administering to the patient a therapeutically effective amount of an inhibitor of interleukin-6 (IL-6) in combination with a therapeutically effective amount of A1AT.

In some embodiments, the IL-6 inhibitor is tocilizumab.

In some embodiments, the composition is administered intravenously.

In some embodiments, tocilizumab can be administered in an amount of about 4 mg/kg to about 8 mg/kg, preferably in an amount of about 5 mg/kg to about 7 mg/kg, at a dose of 400 mg/dose to 800 mg/dose, preferable between 500 mg/dose and 700 mg/dose.

In some embodiments, A1AT can be administered in an amount between 60 mg/kg and 200 mg/kg, preferably between 80 mg/kg and 160 mg/kg, most preferably 120 mg/kg.

The composition of the present invention can be administered at least every 6 hours, or at least every 8 hours, or at least every 12 hours, or at least every 24 hours, or at least every 48 hours, or at least every 72 hours, or at least once a week, or at least once every two weeks. Preferably, the composition is administered twice, one week after the first administration. It is also contemplated that the composition of the present invention is administered as a single dose.

### DETAILED DESCRIPTION

### Definitions

As used herein, the section headings are for organizational purposes only and are not to be construed as limiting the described subject matter in any way. All literature and similar materials cited in this application, including but not limited to, patents, patent applications, articles, books, treatises, and internet web pages are expressly incorporated by reference in their entirety for any purpose. When definitions of terms in incorporated references appear to differ from the definitions provided in the present teachings, the definition provided in the present teachings shall control. It will be appreciated that there is an implied "about" prior to the temperatures, concentrations, times, etc. discussed in the present teachings, such that slight and insubstantial deviations are within the scope of the present teachings herein.

In this application, the use of the singular includes the plural unless specifically stated otherwise. Also, the use of "comprise", "comprises", "comprising", "contain", "contains", "containing", "include", "includes", and "including" are not intended to be limiting.

As used in this specification and claims, the singular forms "a," "an" and "the" include plural references unless the content clearly dictates otherwise.

As used herein, "about" means a quantity, level, value, number, frequency, percentage, dimension, size, amount, weight or length that varies by as much as 20, 15, 10, 9, 8, 7, 6, 5, 4, 3, 2 or 1 % to a reference quantity, level, value, number, frequency, percentage, dimension, size, amount, weight or length.

The term "treatment" or "treating" means any treatment of a disease or disorder in a subject, such as a mammal, including: preventing or protecting against the disease or disorder, that is, causing the clinical symptoms not to develop; inhibiting the disease or disorder, that is, arresting or suppressing the development of clinical symptoms; and/or relieving the disease or disorder that is, causing the regression of clinical symptoms.

It will be understood by those skilled in the art that in human medicine, it is not always possible to distinguish between "preventing" and "suppressing" since the ultimate inductive event or events may be unknown, latent, or the patient is not ascertained until well after the occurrence of the event or events. Therefore, as used herein the term "prophylaxis" is intended as an element of "treatment" to encompass both "preventing" and "suppressing" as defined herein.

The term "therapeutically effective amount" refers to that amount of tocilizumab or A1AT, typically delivered as pharmaceutical compositions, that is sufficient to effect treatment, as defined herein, when administered to a subject in need of such treatment.

Although this disclosure is in the context of certain embodiments and examples, those skilled in the art will understand that the present disclosure extends beyond the specifically disclosed embodiments to other alternative embodiments and/or uses of the embodiments and obvious modifications and equivalents thereof. In addition, while several variations of the embodiments have been shown and described in detail, other modifications, which are within the scope of this disclosure, will be readily apparent to those of skill in the art based upon this disclosure.

It is also contemplated that various combinations or sub-combinations of the specific features and aspects of the embodiments may be made and still fall within the scope of the disclosure. It should be understood that various features and aspects of the disclosed embodiments can be combined with, or substituted for, one another in order to form varying modes or embodiments of the disclosure. Thus, it is intended that the scope of the present disclosure herein disclosed should not be limited by the particular disclosed embodiments described above.

It should be understood, however, that this description, while indicating preferred embodiments of the disclosure, is given by way of illustration only, since various changes and modifications within the spirit and scope of the disclosure will become apparent to those skilled in the art.

The terminology used in the description presented herein is not intended to be interpreted in any limited or restrictive manner. Rather, the terminology is simply being utilized in conjunction with a detailed description of embodiments of the systems, methods and related components. Furthermore, embodiments may comprise several novel features, no single one of which is solely responsible for its desirable attributes or is believed to be essential to practicing the embodiments herein described.

The present disclosure provides methods and compositions for the treatment of CRS in a patient in need thereof. In some embodiments, the method comprises administering to the patient a therapeutically effective amount of tocilizumab in combination with a therapeutically effective amount of A1AT.

In some embodiments, the CRS can be mild, moderate or severe.

In some embodiments, the CRS is caused by the SARS-COV-2 virus.

In some embodiments, the composition comprising tocilizumab and A1AT is administered intravenously.

In some embodiments, tocilizumab can be administered in an amount of about 4 mg/kg to about 8 mg/kg, preferably in an amount of about 5 mg/kg to about 7 mg/kg, at a dose of 400 mg/dose to 800 mg/dose, preferable between 500 mg/dose and 700 mg/dose.

In some embodiments, A1AT can be administered in an amount between 40 mg/kg and 80 mg/kg, preferably between 50 mg/kg and 70 mg/kg, most preferably 60 mg/kg.

The composition of the present invention can be administered at least every 6 hours, or at least every 8 hours, or at least every 12 hours, or at least every 24h, or at least every 48 hours, or at least every 72 hours, or at least once a week, or at least once every two weeks. It is also contemplated that the composition of the present invention is administered as a single dose.

### Alpha-1-antitrypsin

Alpha-1-proteinase inhibitor, also known as Alpha-1-Antitrypsin (A1AT) or α1-Antitrypsin, is a proteinase inhibitor that acts on a variety of cellular proteases. A1PI plays a major role in tissue homeostasis through the inhibition of neutrophil elastase action, and through other mechanisms. A.

Congenital deficiencies of A1PI allow uncontrolled activity of neutrophil elastase and the subsequent degradation of elastin, an essential protein that confers elasticity to tissues, particularly the lungs. The absence of elastin may result in respiratory complications such as pulmonary emphysema and hepatic cirrhosis. Chronic intravenous (IV) administration of A1PI is usually used for the treatment of AAT deficiency.

A1PI can be obtained from human plasma, using the Cohn Fractionation Process, as it is known to the skilled person. However, the A1PI can be obtained from other sources and using different techniques, such as recombinant DNA technique. One suitable example of a pharmaceutical product of A1PI is commercialized under the trade name Prolastin-C (Grifols S.A., Spain).

### Tocilizumab

Tocilizumab (TCZ), is a recombinant humanized, anti-human monoclonal antibody of the immunoglobulin G1k subclass directed against soluble and membrane-bound interleukin 6 receptors (IL-6R). It is marketed in the European Union (EU) under the trade name RoActemra and in the United States as Actemra (Hoffmann-La Roche, Switzerland). TCZ was first approved in 2005 as an orphan drug in Japan for the treatment of Castleman's disease, a rare lymphoproliferative disease involving expansion of plasma cell numbers. TCZ is now licensed in the EU for use alone or in combination with disease-modifying anti-rheumatic drugs (DMARDs) to treat adult patients with moderate to severely active rheumatoid arthritis (RA), children over 2 y of age with the systemic form of juvenile idiopathic arthritis (sJIA) or children over 2 y of age with the polyarticular form of Juvenile Idiopathic Arthritis (pJIA). It has also been studied as a treatment of other conditions such as Crohn's disease, systemic lupus erythematosus, Takayasu Arteritis (TA), Giant Cell Arteritis (GCA) Polymyalgia Rheumatica (PMR) and refractory adult-onset still disease.

### EXAMPLE

### Example 1. Simulation of the predicted effect of the method of the present invention on CRS

To identify new indications of A1AT in combination with other drugs a prediction technology was used. This technology, which is known to the skilled person, creates mathematical models that integrate biochemical, pharmaceutical, and clinical data with the aim of simulating human physiology. The analysis of these models provides mechanistic insight into biological processes and has the potential to uncover non-obvious patterns and molecular explanations.

In this case, a repositioning screening was performed, where the efficacy of combinations of A1AT with other approved drugs (aprox 3000 drugs; source Drugbank database) were computationally predicted by calculating their molecular impact on a set of mathematical models simulating different indications.

A1AT was molecularly characterized: the main proteins, including known A1AT targets and off-targets mediating the known downstream effect of the A1AT.

Once the mathematical models were generated, a drug repositioning analysis was performed on them in order to calculate the efficacy of each combination of A1AT with an existing approved drug for treating each disease. Approximately, 3000 drug combinations were tested on each indication. They were ranked according to the degree of predicted modulation of the indication (100 - 0 %). From all the 3000 combinations made, the higher effect (95 %) on the treatment of CRS was obtained with A1AT in combination with tocilizumab, which is higher than the predicted effect for each of these drugs separately, i.e. 67 % and 47 %, respectively.

The following table shows the predicted effect on Cytokyne Release Syndrome of each drug separately and in combination.

| Drug A | Drug B | Predicted effect of Drug A | Predicted effect of Drug B | Predicted effect of Drug A + Drug B |
|---|---|---|---|---|
| Tocilizumab | A1AT | 47% | 67% | **95 %** |
| Esmolol | A1AT | 76% | 67% | 78 % |
| Lutropin α | A1AT | 78 % | 67 % | 79 % |
| Cimetidine | A1AT | 74 % | 67 % | 79 % |

## Claims

1. Method for the treatment of Cytokine Release Syndrome (CRS) in a patient in need thereof, comprising administering to the patient a therapeutically effective amount of an inhibitor of interleukin-6 (IL-6) in combination with a therapeutically effective amount of human α-1-proteinase inhibitor (A1AT)

2. Method, according to claim 1, wherein the IL-6 inhibitor is tocilizumab.

3. Method, according to claim 1 or 2, wherein the CRS is mild, moderate or severe.

4. Method, according to any of the preceding claims, wherein the CRS is caused by a viral infection, or by a bacterial infection, or by a fungal infection, or by a protozoal infection, or by a combination thereof.

5. Method, according to claim 4, wherein the CRS is caused by the SARS-COV-2 virus.

6. Method, according to any of the preceding claims, wherein said composition is administered intravenously.

7. Method, according to any of the preceding claims, wherein tocilizumab is administered in an amount of about 4 mg/kg to about 8 mg/kg.

8. Method, according to claim 7, wherein tocilizumab is administered in an amount of about 5 mg/kg to about 7 mg/kg.

9. Method, according to any of the preceding claims, wherein tocilizumab is administered at a dose between 400 mg/dose to 800 mg/dose.

10. Method, according to claim 9, wherein tocilizumab is administered at a dose between 500 mg/dose and 700 mg/dose.

11. Method, according to any of the preceding claims, wherein A1AT is administered in an amount between 60 mg/kg and 200 mg/kg.

12. Method, according to claim 11, wherein A1AT is administered in an amount between 80 mg/kg and 160 mg/kg.

13. Method, according to claim 12, wherein A1AT is administered in an amount of 120 mg/kg.

14. Method, according to any of the preceding claims, wherein the composition is administered at least every 6 hours, or at least every 8 hours, or at least every 12 hours, or at least every 24h, or at least every 48 hours, or at least every 72 hours, or at least once a week, or at least once every two weeks.

15. Method, according to any of the preceding claims, wherein the composition is administered twice, one week after the first administration.

16. Method, according to any of the preceding claims, where the composition is administered as a single dose.

17. Composition comprising a therapeutically effective amount of an inhibitor of interleukin-6 and a therapeutically effective amount of human α-1-proteinase inhibitor (A1AT) for the treatment of Cytokine Release Syndrome (CRS) in a patient in need thereof.

18. Composition for use, according to claim 17, wherein the IL-6 inhibitor is tocilizumab.

19. Composition for use, according to claim 17 or 18, wherein the CRS is mild, moderate or severe.

20. Composition for use, according to any of claims 17 to 19, wherein the CRS is caused by a viral infection, or by a bacterial infection, or by a fungal infection, or by a protozoal infection, or by a combination thereof.

21. Composition for use, according to claim 20, wherein the CRS is caused by the SARS-COV-2 virus.

22. Composition for use, according to any of claims 17-21, wherein said composition is administered intravenously.

23. Composition for use, according to any of claims 17-22, wherein tocilizumab is administered in an amount of about 4 mg/kg to about 8 mg/kg.

24. Composition for use, according to claim 23, wherein tocilizumab is administered in an amount of about 5 mg/kg to about 7 mg/kg.

25. Composition for use, according to any of claims 17-24, wherein tocilizumab is administered at a dose between 400 mg/dose to 800 mg/dose.

26. Composition for use, according to claim 25, wherein tocilizumab is administered at a dose between 500 mg/dose and 700 mg/dose.

27. Composition for use, according to any of claims 17-26, wherein A1AT is administered in an amount between 60 mg/kg and 200 mg/kg.

28. Composition for use, according to claim 27, wherein A1AT is administered in an amount between 80 mg/kg and 160 mg/kg.

29. Composition for use, according to claim 28, wherein A1AT is administered in an amount of 120 mg/kg.

30. Composition for use, according to any of claims 17-29, wherein said composition is administered at least every 6 hours, or at least every 8 hours, or at least every 12 hours, or at least every 24h, or at least every 48 hours, or at least every 72 hours, or at least once a week, or at least once every two weeks.

31. Composition for use, according to any of claims 17-29, wherein the composition is administered twice, one week after the first administration.

32. Composition for use, according to any of claims 17-29, wherein the composition is administered as a single dose.

33. Method for the treatment of Acute Respiratory Distress Syndrome (ARDS) resulting from CRS, the method comprises administering to the patient a therapeutically effective amount of an inhibitor of interleukin-6 (IL-6) in combination with a therapeutically effective amount of A1AT.

34. Method for the treatment of Septic Shock, the method comprises administering to the patient a therapeutically effective amount of an inhibitor of interleukin-6 (IL-6) in combination with a therapeutically effective amount of A1AT.

35. Composition a therapeutically effective amount of an inhibitor of interleukin-6 (IL-6) in combination with a therapeutically effective amount of A1AT for use in the treatment of Acute respiratory Distress Syndrome (ARDS).

36. Composition a therapeutically effective amount of an inhibitor of interleukin-6 (IL-6) in combination with a therapeutically effective amount of A1AT for use in the treatment of Septic Shock.
